# EUROPEAN PATENT APPLICATION

(11) **EP 2 529 766 A2**
(43) Date of publication of application: **05.12.2012**
(21) Application number: 12170250.0
(22) Date of filing: 31.05.2012
(51) Int. Cl.: A61M 1/00, A61M 39/10

(54) **Wound drainage equipment, conduit, connector and wound cover**

(30) Priority: 01.06.2011 TW 100119180; 18.04.2012 TW 101113826
(71) Applicant: Suzric Enterprise Co., Ltd, Guishan Township, Taoyuan 333 (TW)
(72) Inventor: Wang, Suzanne, Guishan Township 333 Taoyuan County (TW); Huang, Cheng-Tien, Pingzhen City 324 Taoyuan County (TW)
(74) Representative: Ouzman, Beverley Nicola Claire

(57) **Abstract**

A wound drainage equipment (200) adapted for sucking exudates in a wound is provided. The wound drainage equipment includes a wound drainage assembly and at least one aeration hole (H1). The wound drainage assembly includes a wound cover and a conduit. The wound cover (210) is adapted for being disposed at the wound. The conduit (230) has a lumen adapted for communicating with the wound. The exudates in the wound are adapted for being sucked through the lumen. The aeration hole is disposed at the conduit (230). The aeration hole communicates with the lumen and an external environment. The efficiency of sucking the exudates in the wound according to the wound drainage equipment of an embodiment of the present invention is relatively high.

## Description

### Field of the Invention

The present invention relates to a drainage equipment, and more particularly, to a wound drainage equipment, and a conduit, a connector and a wound cover applied to the wound drainage equipment.

### Background of the Invention

Vacuum assisted closure therapy or negative pressure wound therapy is a method of auxiliary physical therapy. The therapy is carried out by employing a negative pressure pump together with an adhesive drape or a wound cover and by using a porous screen with biocompatibility as a biological dressing in order to create a negative pressure environment inside a wound. The applied negative pressure causes the porous screen to shrink and thus indirectly causes the wound to contract inward. Accordingly, boundary cells of the wound are pulled by negative pressure through the porous screen such that cell division and cell proliferation are enhanced and the healing of the wound can be speeded up. The negative pressure is transmitted within the wound through the porous screen so as to induce interstitial tissue fluid to flow, indirectly promoting blood vessel proliferation and local blood circulation, and draining exudates and pus from the wound. Accordingly, the phenomenon of edema is prevented, the likelihood of inflammation is reduced, a healing environment with appropriate humidity is provided, the wound is protected, and the time period required for the healing of the wound is shortened.

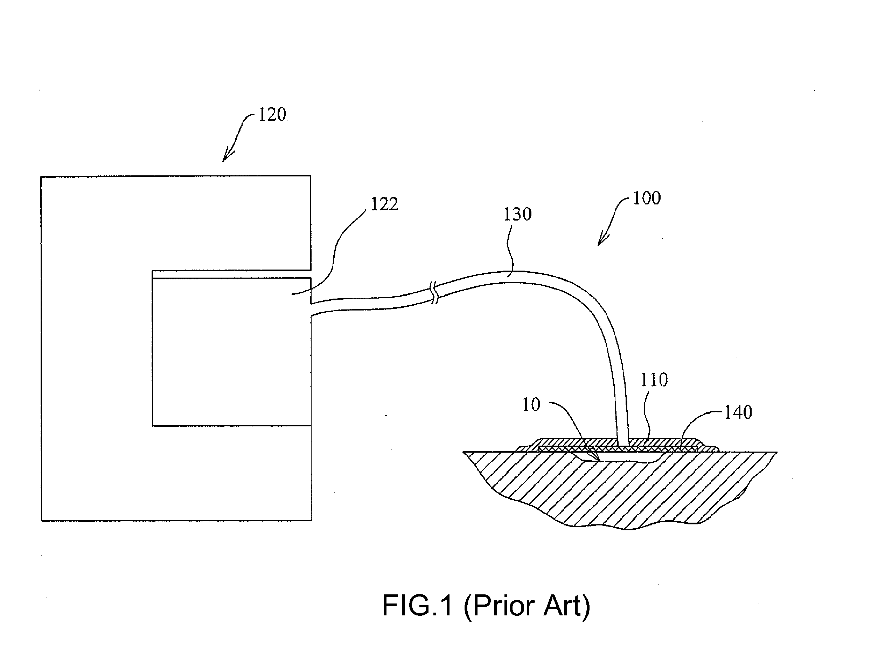

FIG. 1 is a schematic view of a conventional wound drainage equipment. Referring to FIG. 1, a conventional wound drainage equipment 100 comprises a wound cover 110, a suction appliance 120, a conduit 130, and a porous screen 140. The porous screen 140 is disposed at a wound 10. The wound cover 110 is disposed on the porous screen 140 and the wound 10. The suction appliance 120 has a collecting container 122 and a negative pressure pump (not shown). The conduit 130 communicates with the wound 10 and the collecting container 122 of the suction appliance 120. When the wound drainage equipment 100 operates, the negative pressure pump provides a negative pressure, such that exudates in the wound 10 are drained to the collecting container 122 by means of the conduit 130.

However, after the conventional wound drainage equipment 100 operates for a period of time, a virtually hermetical phenomenon is made between the wound cover 110 and the wound 10 and the internal pressure between them is reduced accordingly, and therefore, the efficiency of sucking the exudates in the wound 10 decreases. Accordingly, it is necessary to improve the conventional wound drainage equipment 100.

### Summary of the Invention

The present invention provides an improved wound drainage equipment that can be used to suck exudates in a wound more efficiently.

The present invention provides a wound drainage equipment adapted for sucking exudates in a wound. The wound drainage equipment comprises a wound drainage assembly and at least one aeration hole. The wound drainage assembly comprises a wound cover and a conduit. The wound cover is an adhesive drape, a transparent film, or any substance for covering a wound. The wound cover is adapted for being disposed on the wound. The conduit has a lumen adapted for communicating with the wound. The exudates in the wound are sucked through the lumen. The aeration hole is disposed at the conduit. The aeration hole communicates with the lumen of the conduit and the external environment.

The aeration hole can penetrate through a wall of the conduit so as to be connected to the lumen.

The conduit can comprise a first tube body, a second tube body and a connector. The connector is adapted for connecting the first tube body and the second tube body. The first tube body is adapted for communicating with the wound.

The aeration hole can be disposed at the connector.

The wound drainage assembly can further comprise a suction appliance. The lumen of the conduit is adapted for communicating with the wound and the suction appliance. The suction appliance is adapted for sucking the exudates in the wound through the lumen.

The suction appliance can comprise a collecting container adapted for collecting the exudates from the wound. The lumen of the conduit is adapted for communicating with the wound and the collecting container.

The present invention also provides a wound drainage equipment adapted for sucking exudates in a wound. The wound drainage equipment comprises a wound drainage assembly and at least one aeration hole. The wound drainage assembly comprises a wound cover and a conduit. The wound cover is adapted for being disposed on the wound and comprises a first connection hole and a second connection hole. The second connection hole faces the wound. The conduit has a lumen adapted for communicating with the wound. The exudates in the wound are sucked through the lumen, and one end of the conduit is adapted for being disposed at the first connection hole such that the first connection hole communicates with the lumen. The aeration hole is disposed at the wound cover. The aeration hole communicates with the lumen and the external environment. An outer radius of an opening of the aeration hole is smaller than an outer radius of an opening of the first connection hole. A direction of the opening of the aeration hole is substantially parallel to a direction of the opening of the first connection hole. The direction of the opening of the aeration hole is substantially not parallel to a direction of an opening of the second connection hole. The distance between a center of the opening of the aeration hole and a center of the opening of the first connection hole is smaller than or equal to the sum of the outer radius of the opening of the aeration hole and the outer radius of the opening of the first connection hole. The distance between the center of the opening of the aeration hole and a plane where the opening of the second connection hole is located is smaller than the distance between the center of the opening of the first connection hole and the plane where the opening of the second connection hole is located.

Optionally, the aeration hole communicates with the first connection hole only through an inner space of the wound cover.

The wound cover can further comprise an injection hole. An outer radius of an opening of the injection hole is smaller than the outer radius of the opening of the first connection hole. A direction of the opening of the injection hole is substantially parallel to the direction of the opening of the first connection hole. The distance between a center of the opening of the injection hole and the center of the opening of the first connection hole is smaller than or equal to the sum of the outer radius of the opening of the injection hole and the outer radius of the opening of the first connection hole. The distance between the center of the opening of the injection hole and the plane where the opening of the second connection hole is located is smaller than the distance between the center of the opening of the first connection hole and the plane where the opening of the second connection hole is located.

Optionally, the wound drainage assembly further comprises an injection tube, and one end of the injection tube is adapted for being disposed at the injection hole.

The wound drainage assembly can further comprise an aeration tube, and one end of the aeration tube is adapted for being disposed at the aeration hole.

The present invention also provides a conduit applicable to a wound drainage equipment adapted for sucking exudates from a wound. The conduit comprises a lumen and at least one aeration hole. The lumen is adapted for communicating with the wound. The exudates in the wound are adapted for being sucked through the lumen. The aeration hole communicates with the lumen and an external environment.

The aeration hole can penetrate through a wall of the conduit so as to be connected to the lumen.

The conduit can have a first tube body, a second tube body and a connector. The connector is adapted for connecting the first tube body and the second tube body. The first tube body is adapted for communicating with the wound.

The aeration hole can be disposed at the connector.

The present invention also provides a connector adapted for being connected to a tube body. The connector and the tube body are applicable to a wound drainage equipment adapted for sucking exudates in a wound. The tube body is adapted for communicating with the wound. The connector comprises a lumen and at least one aeration hole. The lumen is adapted for communicating with the tube body. The exudates in the wound are sucked through the lumen and the tube body. The aeration hole communicates with the lumen of the conduit and the external environment.

The aeration hole can penetrate through a wall of the connector so as to be connected to the lumen.

The present invention also provides a wound cover adapted for being disposed on a wound and can be used with a wound drainage equipment adapted for sucking exudates in the wound. The wound cover comprises a first connection hole, a second connection hole and at least one aeration hole. The first connection hole communicates with the wound and is adapted to be connected to a conduit of the wound drainage equipment. The exudates in the wound are sucked through the first connection hole and a lumen of the conduit. The second connection hole faces the wound. The aeration hole communicates with the lumen and the external environment. An outer radius of an opening of the aeration hole is smaller than an outer radius of an opening of the first connection hole. A direction of the opening of the aeration hole is substantially parallel to a direction of the opening of the first connection hole. The direction of the opening of the aeration hole is substantially not parallel to a direction of an opening of the second connection hole. The distance between a center of the opening of the aeration hole and a center of the opening of the first connection hole is smaller than or equal to the sum of the outer radius of the opening of the aeration hole and the outer radius of the opening of the first connection hole. The distance between the center of the opening of the aeration hole and a plane where the opening of the second connection hole is located is smaller than the distance between the center of the opening of the first connection hole and the plane where the opening of the second connection hole is located.

Optionally, the aeration hole communicates with the first connection hole only through an inner space of the wound cover.

The wound cover can further comprise an injection hole. An outer radius of an opening of the injection hole is smaller than the outer radius of the opening of the first connection hole. A direction of the opening of the injection hole is substantially parallel to the direction of the opening of the first connection hole. The distance between a center of the opening of the injection hole and the center of the opening of the first connection hole is smaller than or equal to the sum of the outer radius of the opening of the injection hole and the outer radius of the opening of the first connection hole. The distance between the center of the opening of the injection hole and the plane where the opening of the second connection hole is located is smaller than the distance between the center of the opening of the first connection hole and the plane where the opening of the second connection hole is located.

The wound drainage equipment of the present invention has the following advantage. The aeration hole communicates with the lumen of the conduit and the external environment, and therefore, when the suction appliance drains exudates in a wound, an airflow is admitted into the lumen via the aeration hole which drives exudates in the lumen to flow from the wound toward the suction appliance. Hence, compared with the prior art, in the course of operation of the wound drainage equipment of the present invention, the exudates in the lumen of the conduit flow better such that the wound drainage equipment of the present invention sucks the exudates in the wound more efficiently.

Preferred or alternative features of each aspect or embodiment of the invention apply *mutatis mutandis* to each other embodiment of the invention, unless the context demands otherwise.
The aforesaid features and advantages of the present invention are further illustrated with the following description and the appended claims or the embodiments described hereunder.

### Brief Description of the Drawings

FIG. 1 is a schematic view of a conventional wound drainage equipment.

FIG. 2A is a schematic view of a wound drainage equipment according to a first embodiment of the present invention.

FIG. 2B is a cross-sectional schematic view of a conduit taken along line EE of FIG. 2A.

FIG. 3 is an exploded schematic view of a conduit according to a second embodiment of the present invention.

FIG. 4 is a schematic view of a wound drainage equipment according to a third embodiment of the present invention.

FIG. 5 is a schematic view of a wound drainage equipment according to a fourth embodiment of the present invention.

FIG. 6A is a three-dimensional schematic view of a wound cover according to a fifth embodiment of the present invention.

FIG. 6B is a top schematic view of the wound cover of FIG. 6A.

FIG. 6C is a cross-sectional schematic view of the wound cover taken along line FF of FIG. 6B.

FIG. 6D is a cross-sectional schematic view of the wound cover of FIG. 6C where the conduit and the aeration tube are disposed.

FIG. 6E is a three-dimensional schematic view of the wound cover of FIG. 6A where the conduit and the aeration tube are disposed.

FIG. 7A is a three-dimensional schematic view of a wound cover according to a sixth embodiment of the present invention.

FIG. 7B is a three-dimensional schematic view of the wound cover of FIG. 7A where the conduit, the aeration tube, and the injection tube are disposed.

### Detailed Description of the Invention

The preferred embodiments of the present invention will now be described in greater detail by referring to the drawings that accompany the present application. It should be noted that the features illustrated in the drawings are not necessarily drawn to scale. Descriptions of well-known components, materials, and process techniques are omitted so as to not unnecessarily obscure the embodiments of the invention. Any devices, components, materials, and steps described in the embodiments are only for illustration and not intended to limit the scope of the present invention.

[First Embodiment]

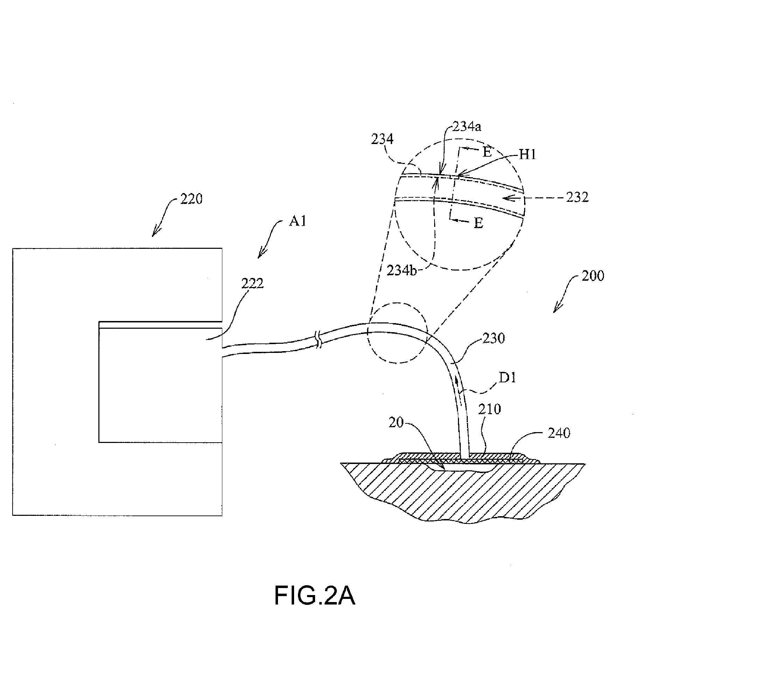

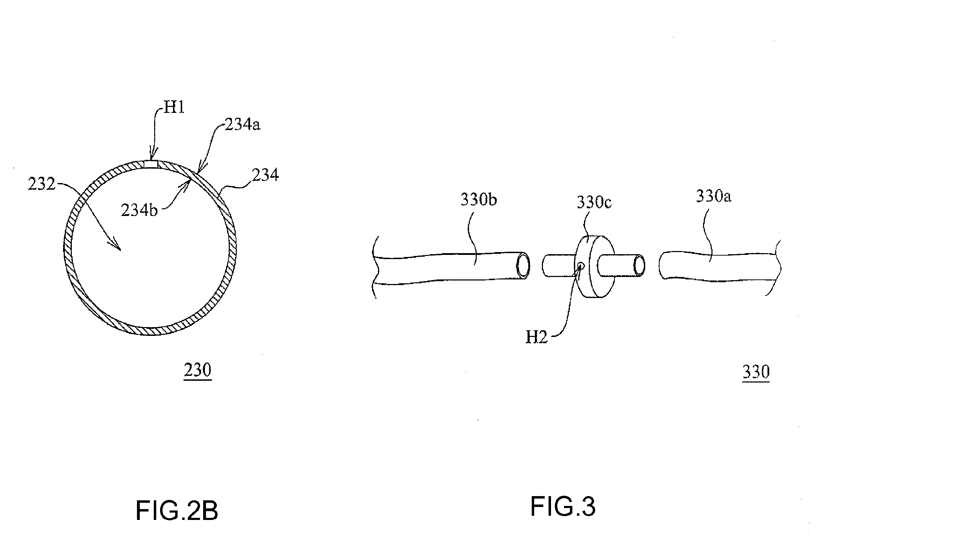

FIG. 2A is a schematic view of a wound drainage equipment according to the first embodiment of the present invention. FIG. 2B is a cross-sectional schematic view of a conduit taken along line EE of FIG. 2A. Referring to FIG. 2A and FIG. 2B, a wound drainage equipment 200 in this embodiment is adapted for sucking exudates from a wound 20. The wound drainage equipment 200 comprises a wound drainage assembly A1 and at least one aeration hole H1. The wound drainage assembly A1 comprises a wound cover 210, a suction appliance 220, a conduit 230, and a porous screen 240.

The porous screen 240 is adapted for being disposed at the wound 20. The wound cover 210 is adapted for being disposed on the porous screen 240 and the wound 20. In this embodiment, the porous screen 240 is a foam-style substance which is made of polyurethane (PU) or polyvinyl alcohol (PVA) and is capable of absorbing water and has a porous structure. The porous screen 240 can also be gauze. In this embodiment, the wound cover 210 is an adhesive film, and the periphery of the adhesive film is affixed to the human skin in the vicinity of the wound 20. The wound cover 210 in this embodiment may be a cup-shaped cover but is not shown in the figures.

The suction appliance 220 has a collecting container 222 and a negative pressure pump (not shown). The conduit 230 has a lumen 232 which is adapted for communicating with the wound 20 and the collecting container 222 of the suction appliance 220. In this embodiment, the conduit 230 penetrates the wound cover 210 such as an adhesive film to come into contact with the porous screen 240.

The aeration hole H1 is disposed at the conduit 230 of the wound drainage assembly A1. The aeration hole H1 communicates with the lumen 232 and an external environment. To be specific, the aeration hole H1 of this embodiment extends from an outer surface 234a of a wall 234 of the conduit 230 to an inner surface 234b of the wall 234, so as to be connected to the lumen 232. In this embodiment, although only one said aeration hole H1 is schematically shown in the figures, there can be more than one said aeration hole H1 as needed.

In the course of operation of the wound drainage equipment 200, the negative pressure pump of the suction appliance 220 provides a negative pressure, and the negative pressure is transmitted to the wound 20 via the collecting container 222, the lumen 232 of the conduit 230, and the porous screen 240 such that the exudates are drained from the wound 20 into the collecting container 222 of the suction appliance 220 via the lumen 232 of the conduit 230 and in a flow direction D1 (that is, the flow direction D1 that leaves the wound 20 and heads for the suction appliance 220).

The aeration hole H1 communicates with the lumen 232 of the conduit 230 and the external environment, and therefore, when the suction appliance 220 provides the negative pressure to drain exudates in the wound 20, an airflow will be admitted into the lumen 232 via the aeration hole H1 and then flow in the flow direction D1. Hence, compared with the prior art, in the course of operation of the wound drainage equipment 200 in this embodiment, the fluid in the lumen 232 of the conduit 230 flows better such that the wound drainage equipment 200 in this embodiment sucks the exudates in the wound 20 more efficiently.

[Second Embodiment]

FIG. 3 is an exploded schematic view of a conduit according to a second embodiment of the present invention. Referring to FIG. 3, a conduit 330 of this embodiment can be substituted for the conduit 230 of the first embodiment. The conduit 330 of this embodiment has a first tube body 330a, a second tube body 330b, and a connector 330c. The connector 330c is adapted for connecting the first tube body 330a and the second tube body 330b. An end of the first tube body 330a is opposite to the connector 330c and adapted for communicating with the wound 20 (shown in FIG. 2A). An end of the second tube body 330b is opposite to the connector 330c and adapted to be connected to the collecting container 222 of the suction appliance 220 (shown in FIG. 2A). In this embodiment, an aeration hole H2 is disposed at the connector 330c.

[Third Embodiment]

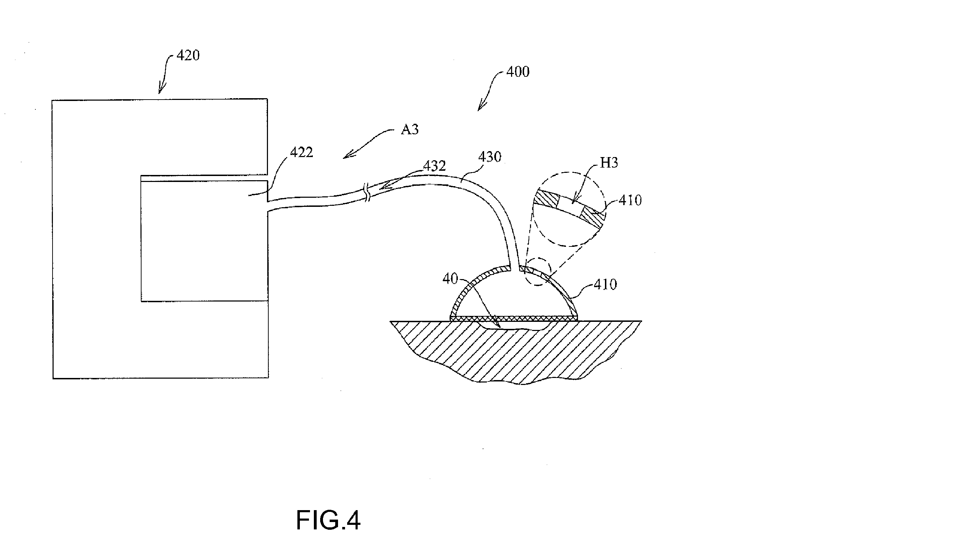

FIG. 4 is a schematic view of a wound drainage equipment according to a third embodiment of the present invention. Referring to FIG. 4, in this embodiment, an aeration hole H3 of wound drainage equipment 400 is disposed at a wound cover 410 of a wound drainage assembly A3. The wound cover 410 is a cup-shaped cover. A lumen 432 of a conduit 430 communicates with the wound cover 410 and a collecting container 422 of a suction appliance 420 and thereby communicates with a wound 40 and the collecting container 422.

[Fourth Embodiment]

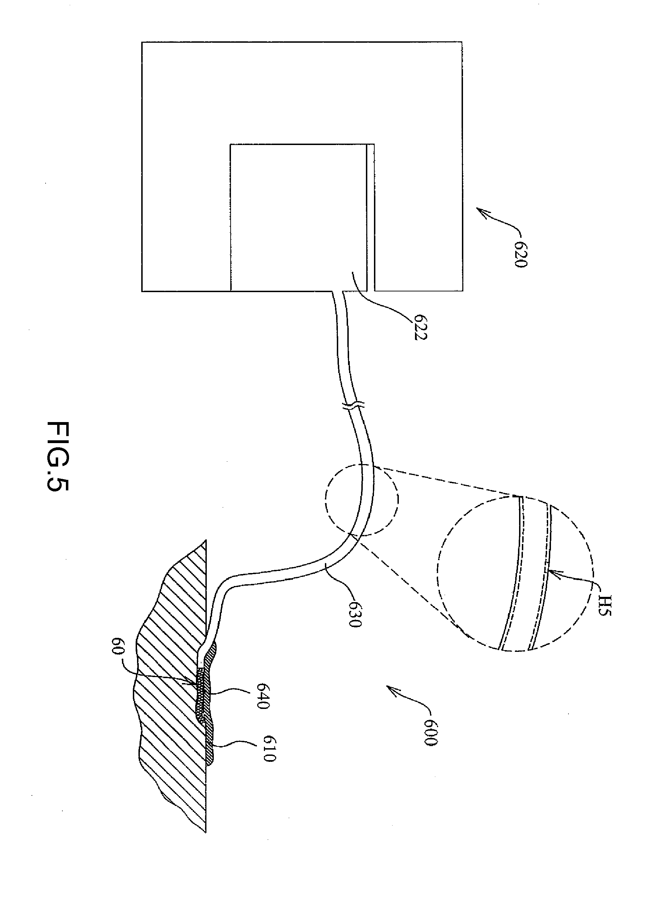

FIG. 5 is a schematic view of a wound drainage equipment according to a fourth embodiment of the present invention. Referring to FIG. 5, in this embodiment, an end of a conduit 630 of a wound drainage equipment 600 is inserted into a wound 60, and a porous screen 640 such as a gauze or a foam or others is filled in the wound 60. Furthermore, the porous screen 640, a portion of the conduit 630, and the wound 60 are covered by a wound cover 610 such as an adhesive film. In this embodiment, an aeration hole H5 is disposed at the conduit 630.

[Fifth Embodiment]

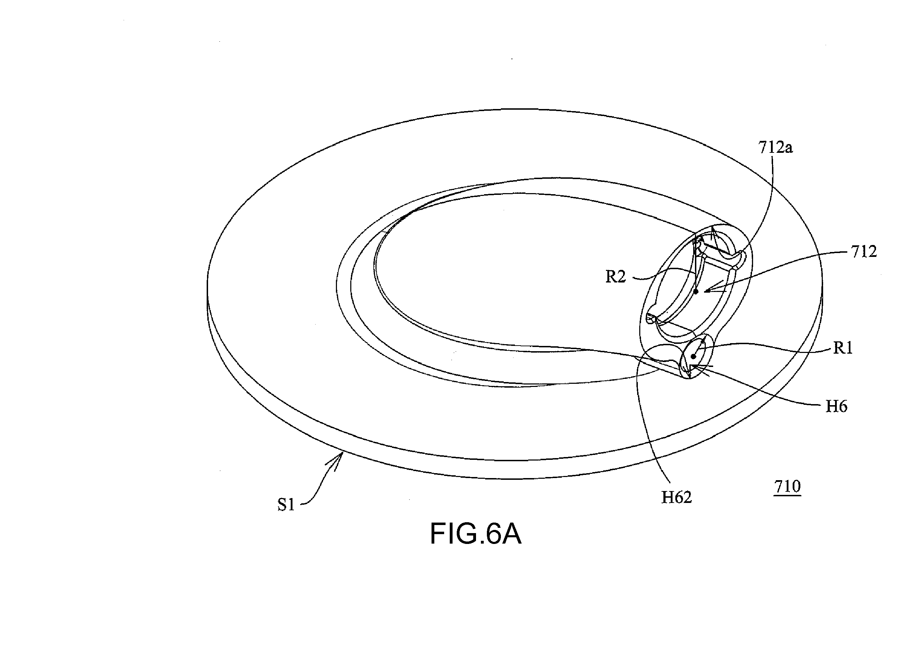

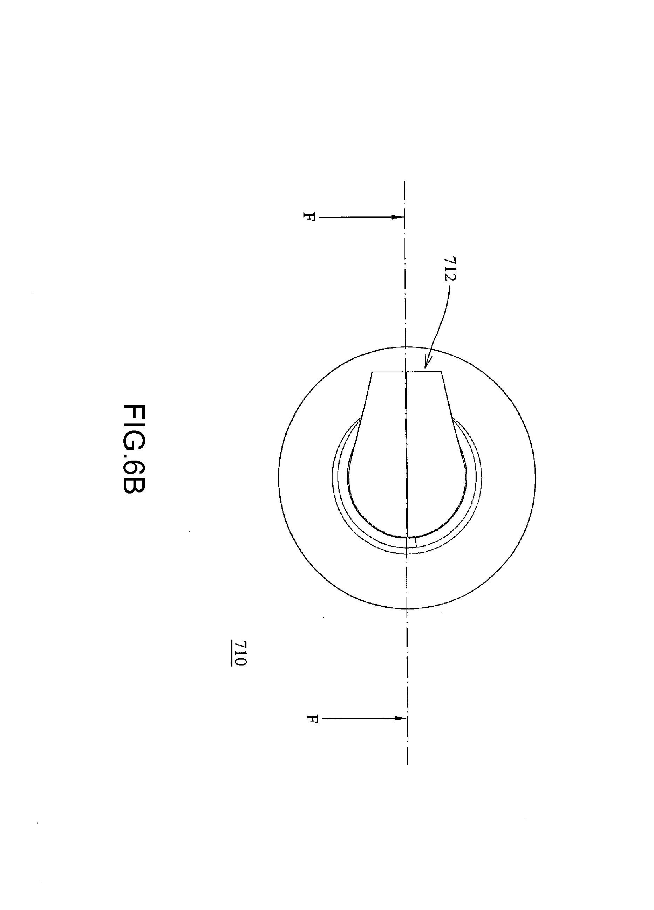

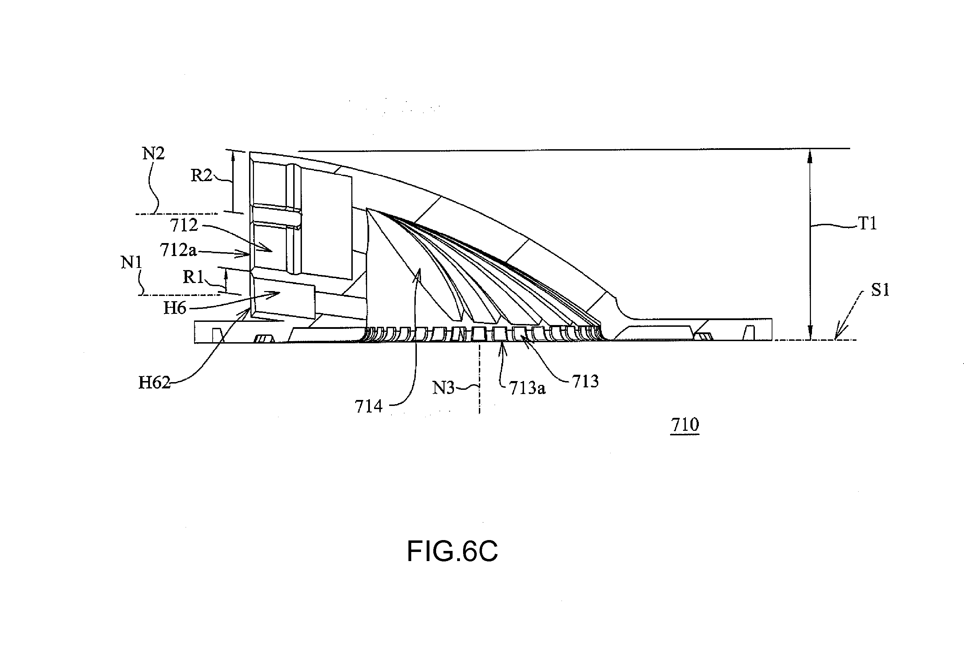

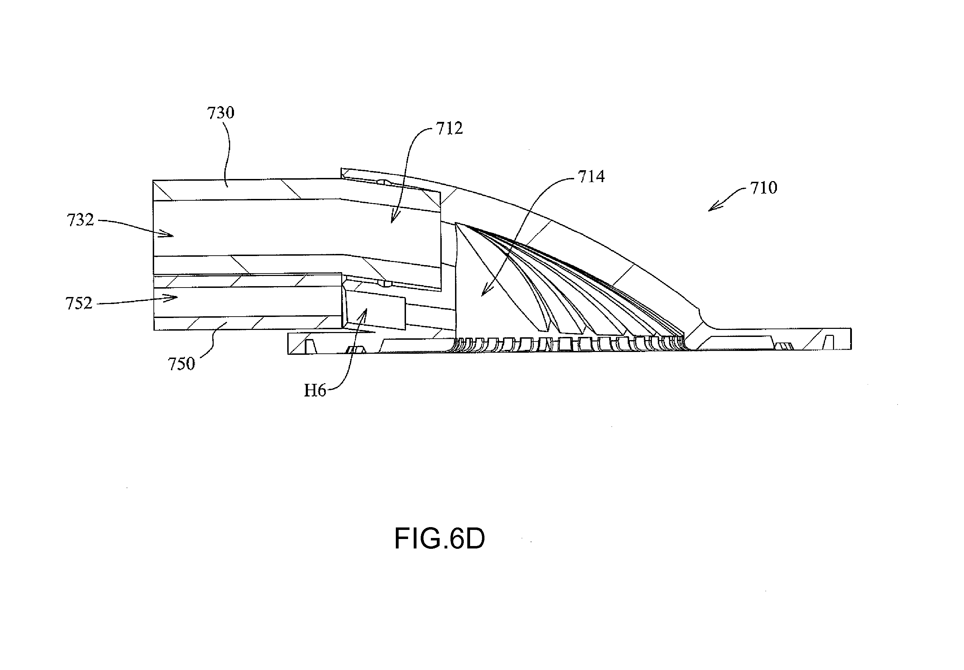

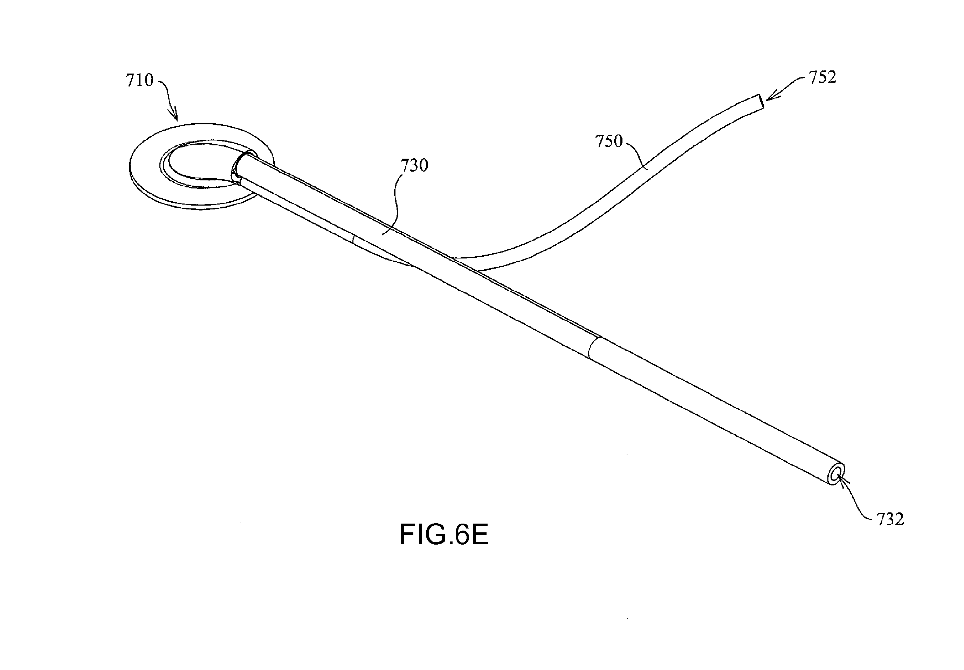

FIG. 6A is a three-dimensional schematic view of a wound cover according to a fifth embodiment of the present invention. FIG. 6B is a top schematic view of the wound cover of FIG. 6A. FIG. 6C is a cross-sectional schematic view of the wound cover taken along line FF of FIG. 6B. FIG. 6D is a cross-sectional schematic view of the wound cover of FIG. 6C where the conduit and the aeration tube are disposed. FIG. 6E is a three-dimensional schematic view of the wound cover of FIG. 6A where the conduit and the aeration tube are disposed. Referring to FIGS. 6A to 6E, the wound cover 710 of the present embodiment can replace the wound cover 410 of the third embodiment so as to be disposed on the wound 40 (see FIG. 4). The wound cover 710 of the present embodiment includes a first connection hole 712 and a second connection hole 713. The aeration hole H6 is disposed at the wound cover 710. The second connection hole 713 is adapted for facing the wound 40 (see FIG. 4). It should be noted that the aeration hole H6 communicates with the first connection hole 712 only through an inner space 714 of the wound cover 710. A filter (not shown) can be disposed at the aeration hole H6.

One end of the conduit 730 is adapted for being disposed at the first connection hole 712 and another end of the conduit 730 is adapted for being connected to a suction appliance (not shown) such that the first connection hole 712 communicates with the lumen 732 of conduit 730 so as to communicate with the suction appliance. It should be emphasized that the aeration hole H6 communicates with the lumen 732 of the conduit 730 and the external environment. In addition, the aeration hole H6 can be optionally connected to an aeration tube 750 such that the aeration hole H6 communicates with the lumen 752 of the aeration tube 750.

In the present embodiment, the aeration hole H6 is located below the first connection hole 712. An outer radius R1 of an opening H62 of the aeration hole H6 is smaller than an outer radius R2 of an opening 712a of the first connection hole 712. A direction N1 of the opening H62 of the aeration hole H6 is substantially parallel to a direction N2 of the opening 712a of the first connection hole 712. The direction N1 of the opening H62 of the aeration hole H6 is substantially not parallel to a direction N3 of an opening 713a of the second connection hole 713. For example, in the present embodiment the direction N1 is almost perpendicular to the direction N3. It should be stated that the term "direction of the opening" means the direction which is normal to the plane in which the opening is located. The distance between a center of the opening H62 of the aeration hole H6 and a center of the opening 712a of the first connection hole 712 is smaller than or equal to the sum of the outer radius R1 of the opening H62 of the aeration hole H6 and the outer radius R2 of the opening 712a of the first connection hole 712. The distance between the center of the opening H62 of the aeration hole H6 and a plane S1 where the opening 713a of the second connection hole 713 is located is smaller than the distance between the center of the opening 712a of the first connection hole 712 and the plane S1. Therefore, the aeration hole H6 can be disposed as closely as possible to the first connection hole 712 such that the height T1 of the wound cover 710 can be as small as possible. Accordingly, a patient using the wound cover 710 can feel more comfortable.

In addition, because the aeration hole H6 can be optionally connected to the aeration tube 750, the aeration function of the aeration hole H6 can be extended by means of the aeration tube 750 such that the aeration hole H6 is not easily pressed by a patient to cause the aeration hole H6 not to function well when the patient uses the wound drainage assembly having the wound cover 710 and the aeration tube 750.

[Sixth Embodiment]

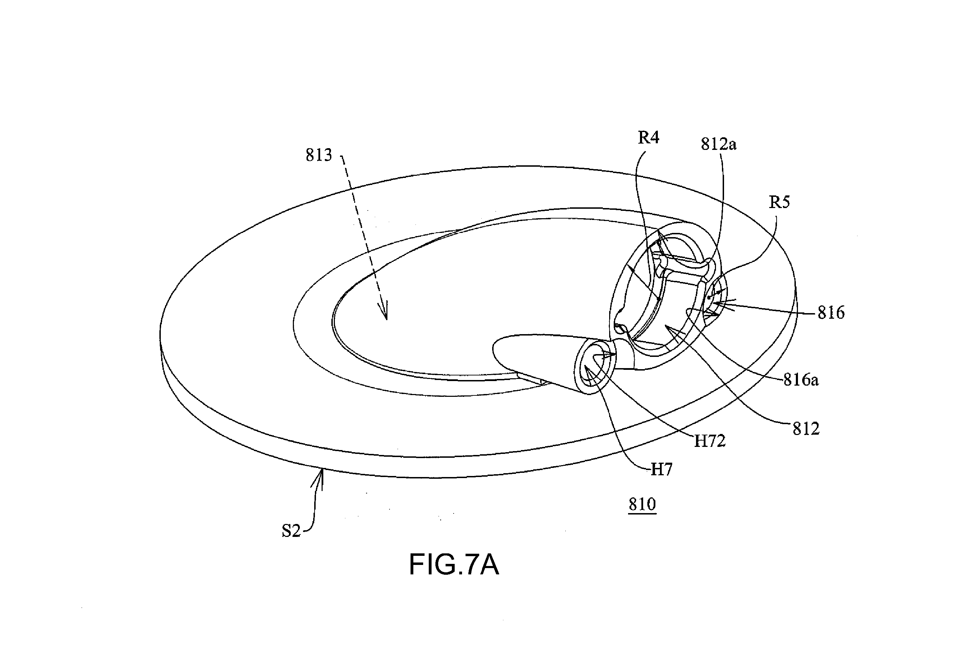

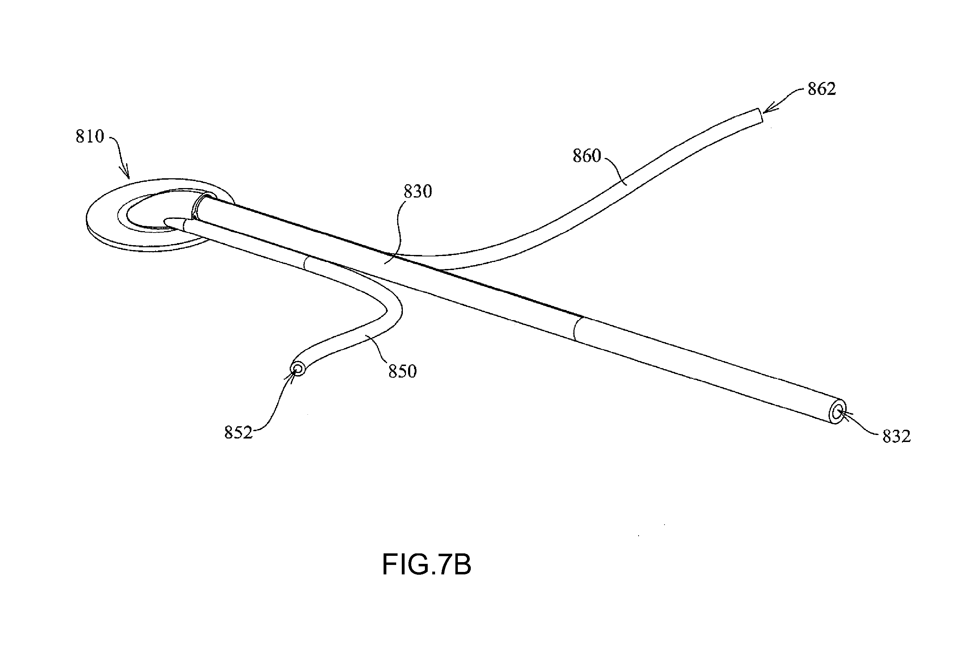

FIG. 7A is a three-dimensional schematic view of a wound cover according to a sixth embodiment of the present invention. FIG. 7B is a three-dimensional schematic view of the wound cover of FIG. 7A where the conduit, the aeration tube, and the injection tube are disposed. Referring to FIG. 7A and FIG. 7B, the difference between the wound cover 810 of the present embodiment and the wound cover 710 of the fifth embodiment is that the wound cover 810 further includes an injection hole 816. The injection hole 816 and the aeration hole H7 are disposed at two sides of the first connection hole 812. It should be noted that the aeration hole H7 communicates with the first connection hole 812 only through an inner space of the wound cover 810.

One end of the conduit 830 is adapted for being disposed at the first connection hole 812 and another end of the conduit 830 is adapted for being connected to a suction appliance (not shown) such that the first connection hole 812 communicates with the lumen 832 of conduit 830 so as to communicate with the suction appliance. In addition, the aeration hole H7 can be optionally connected to an aeration tube 850 such that the aeration hole H7 communicates with the lumen 852 of the aeration tube 850. Furthermore, the injection hole 816 can be optionally connected to an injection tube 860 such that the injection hole 816 communicates with the lumen 862 of the injection tube 860. When a patient uses the wound cover 810 and the injection tube 860, therapeutic liquid can be injected into the wound (not shown) through the injection tube 860 and the injection hole 816.

In the present embodiment, the relationship between the opening H72 of the aeration hole H7, the opening 812a of the first connection hole 812 and the opening of the second connection hole 813 can be referred to the relationship between the opening H62 of the aeration hole H6, the opening 712a of the first connection hole 712 and the opening 713a of the second connection hole 713 of the fifth embodiment, and therefore, any further detailed description is omitted herein. The outer radius R5 of an opening 816a of the injection hole 816 is smaller than the outer radius R4 of the opening 812a of the first connection hole 812. The direction of the opening 816a of the injection hole 816 is substantially parallel to the direction of the opening 812a of the first connection hole 812. The distance between the center of the opening 816a of the injection hole 816 and the center of the opening 812a of the first connection hole 812 is smaller than or equal to the sum of the outer radius R5 of the opening 816a of the injection hole 816 and the outer radius R4 of the opening 812a of the first connection hole 812. The distance between the center of the opening 816a of the injection hole 816 and the plane S2 where the opening of the second connection hole 813 is located is smaller than the distance between the center of the opening 812a of the first connection hole 812 and the plane S2.

The wound drainage equipment according to the present invention has the following advantage. The aeration hole communicates with the lumen of the conduit and an external environment, and therefore, when the suction appliance drains exudates in a wound, an airflow admitted into the lumen via the aeration hole drives fluid in the lumen to flow from the wound towards the suction appliance. Hence, compared with the prior art, in the course of operation of the wound drainage equipment in the embodiment of the present invention, the fluid in the lumen of the conduit flows better, such that the wound drainage equipment of the present invention sucks the exudates in the wound more efficiently.

The foregoing preferred embodiments are provided to illustrate and disclose the technical features of the present invention, and are not intended to be restrictive of the scope of the present invention. Indeed various modifications of the described modes of carrying out the invention which are obvious to those skilled in the art are intended to be covered by the present invention.

## Claims

1. A wound drainage equipment (200, 600) adapted for sucking exudates in a wound (20, 60), comprising:
a wound cover (210, 610) adapted for being disposed on the wound (20, 60); and
a conduit (230, 330, 630) having a lumen (232) adapted for communicating with the wound (20, 60) and at least one aeration hole (H1, H2, H5), wherein the exudates in the wound (20, 60) are sucked through the lumen (232);
wherein the aeration hole (H1, H2, H5) communicates with the lumen (232) and the external environment.

2. The wound drainage equipment (200, 600) of claim 1, wherein the aeration hole (H1, H2, H5) penetrates through a wall (234) of the conduit (230, 330, 630) so as to be connected to the lumen (232).

3. The wound drainage equipment (200, 600) of claim 1 or 2, wherein the conduit (330) has a first tube body (330a), a second tube body (330b) and a connector (330c), the connector (330c) is adapted for connecting the first tube body (330a) and the second tube body (330b), the first tube body (330a) is adapted for communicating with the wound (20, 60), and the aeration hole (H2) is disposed at the connector (330c).

4. A wound drainage equipment (400) adapted for sucking exudates in a wound (40), comprising:
a wound cover (710, 810) adapted for being disposed on the wound (40), comprising a first connection hole (712, 812),a second connection hole (713, 813) and at least one aeration hole (H6, H7), wherein the second connection hole (713, 813) is adapted for facing the wound (40); and
a conduit (730, 830) having a lumen (732, 832) adapted for communicating with the wound (40), wherein the exudates in the wound (40) are sucked through the lumen (732, 832), and one end of the conduit (730, 830) is adapted for being disposed at the first connection hole (712, 812) such that the first connection hole (712, 812) communicates with the lumen (732, 832);
wherein the aeration hole (H6, H7) communicates with the lumen (732, 832) and the external environment, an outer radius (R1) of an opening (H62, H72) of the aeration hole (H6, H7) is smaller than an outer radius (R2, R4) of an opening (712a, 812a) of the first connection hole (712, 812), a direction (N1) of the opening (H62, H72) of the aeration hole (H6, H7) is substantially parallel to a direction (N2) of the opening (712a, 812a) of the first connection hole (712, 812), the direction (N1) of the opening (H62, H72) of the aeration hole (H6, H7) is substantially not parallel to a direction (N3) of an opening (713a) of the second connection hole (713, 813), the distance between a center of the opening (H62, H72) of the aeration hole (H6, H7) and a center of the opening (712a, 812a) of the first connection hole (712, 812) is smaller than or equal to the sum of the outer radius (R1) of the opening (H62, H72) of the aeration hole (H6, H7) and the outer radius (R2, R4) of the opening (712a, 812a) of the first connection hole (712, 812), and the distance between the center of the opening (H62, H72) of the aeration hole (H6, H7) and a plane (S1, S2) where the opening (713a) of the second connection hole (713, 813) is located is smaller than the distance between the center of the opening (712a, 812a) of the first connection hole (712, 812) and the plane (S1, S2) where the opening (713a) of the second connection hole (713, 813) is located.

5. The wound drainage equipment (400) of claim 4, wherein the aeration hole (H6, H7) communicates with the first connection hole (712, 812) only through an inner space (714) of the wound cover (710, 810).

6. The wound drainage equipment (400) of claim 4 or 5, wherein the wound cover (810) further comprises an injection hole (816), an outer radius (R5) of an opening (816a) of the injection hole (816) is smaller than the outer radius (R4) of the opening (812a) of the first connection hole (812), a direction of the opening (816a) of the injection hole (816) is substantially parallel to the direction of the opening (812a) of the first connection hole (812), the distance between a center of the opening (816a) of the injection hole (816) and the center of the opening (812a) of the first connection hole (812) is smaller than or equal to the sum of the outer radius (R5) of the opening (816a) of the injection hole (816) and the outer radius (R4) of the opening (812a) of the first connection hole (812), and the distance between the center of the opening (816a) of the injection hole (816) and the plane (S2) where the opening of the second connection hole (813) is located is smaller than the distance between the center of the opening (812a) of the first connection hole (812) and the plane (S2) where the opening of the second connection hole (813) is located.

7. The wound drainage equipment (400) of claim 6, further comprising an injection tube (860), and one end of the injection tube (860) is adapted for being disposed at the injection hole (816).

8. The wound drainage equipment (400) of any one of claims 4 to 7, further comprising an aeration tube (750, 850), and one end of the aeration tube (750, 850) is adapted for being disposed at the aeration hole (H6, H7).

9. A conduit (230, 330, 630) applicable to a wound drainage equipment (200, 600) of claim 1 adapted for sucking exudates in a wound (20, 60), comprising:
a lumen (232) adapted for communicating with the wound (20, 60), wherein the exudates in the wound (20, 60) are sucked through the lumen (232); and
at least one aeration hole (H1, H2, H5) communicating with the lumen (232) and the external environment.

10. The conduit (230, 330, 630) of claim 9, wherein the aeration hole (H1, H2, H5) penetrates through a wall (234) of the conduit (230, 330, 630) so as to be connected to the lumen (232).

11. The conduit (230, 330, 630) of claim 9 or 10, having a first tube body (330a), a second tube body (330b) and a connector (330c), wherein the connector (330c) is adapted for connecting the first tube body (330a) and the second tube body (330b), the first tube body (330a) is adapted for communicating with the wound (20, 60), and the aeration hole is (H2) disposed at the connector (330c).

12. A connector (330c) adapted for being connected to a tube body; the connector (330c) and the tube body (330a) applicable to a conduit (230, 330, 630) of a wound drainage equipment (200, 600) of claim 3 adapted for sucking exudates in a wound (20, 60), the tube body (330a) adapted for communicating the wound (20, 60), wherein the connector (330c) comprises:
a lumen adapted for communicating with the tube body (330a), wherein the exudates in the wound (20, 60) are sucked through the lumen and the tube body (330a); and
at least one aeration hole (H2) communicating with the lumen and the external environment.

13. The connector (330c) of claim 12, wherein the aeration hole (H2) penetrates through a wall of the connector (330c) so as to be connected to the lumen.

14. A wound cover (710, 810) adapted for being disposed on a wound (40) and applicable to a wound drainage equipment (400) of claim 4 adapted for sucking exudates in the wound (40), comprising:
a first connection hole (712, 812) communicating with the wound (40) and adapted for be connected to a conduit (730, 830) of the wound drainage equipment (400), wherein the exudates in the wound (40) are sucked through the first connection hole (712, 812) and a lumen (732, 832) of the conduit (730, 830);
a second connection hole (713, 813) adapted for facing the wound (40); and
at least one aeration hole (H6, H7) adapted for communicating with the lumen (732, 832) and the external environment, wherein an outer radius (R1) of an opening (H62, H72) of the aeration hole (H6, H7) is smaller than an outer radius (R2, R4) of an opening (712a, 812a) of the first connection hole (712, 812), a direction (N1) of the opening (H62, H72) of the aeration hole (H6, H7) is substantially parallel to a direction (N2) of the opening (712a, 812a) of the first connection hole (712, 812), the direction (N1) of the opening (H62, H72) of the aeration hole (H6, H7) is substantially not parallel to a direction (N3) of an opening (713a) of the second connection hole (713, 813), the distance between a center of the opening (H62, H72) of the aeration hole (H6, H7) and a center of the opening (712a, 812a) of the first connection hole (712, 812) is smaller than or equal to the sum of the outer radius (R1) of the opening (H62, H72) of the aeration hole (H6, H7) and the outer radius (R2, R4) of the opening (712a, 812a) of the first connection hole (712, 812), and the distance between the center of the opening (H62, H72) of the aeration hole (H6, H7) and a plane (S1, S2) where the opening of the second connection hole (713, 813) is located is smaller than the distance between the center of the opening (712a, 812a) of the first connection hole (712, 812) and the plane (S1, S2) where the opening of the second connection hole (713, 813) is located.

15. The wound cover (710, 810) of claim 14, wherein the aeration hole (H6, H7) communicates
with the first connection hole (712, 812) only through an inner space (714) of the wound cover (710, 810).
